# EUROPEAN PATENT SPECIFICATION

(11) **EP 4 445 925 B1**
(45) Date of publication and mention of the grant of the patent: **03.06.2026**
(21) Application number: 23167940.8
(22) Date of filing: 14.04.2023
(51) Int. Cl.: A61M 1/16, A61J 1/14, A61M 39/10

(54) **CONTAINER FOR CONCENTRATE, SYSTEM AND METHOD FOR CONNECTING A CONCENTRATE CONTAINER TO A BLOOD TREATMENT DEVICE**
KONZENTRATBEHÄLTER, SYSTEM UND VERFAHREN ZUM VERBINDEN EINES KONZENTRATBEHÄLTERS MIT EINER BLUTBEHANDLUNGSVORRICHTUNG
RÉCIPIENT DE CONCENTRÉ, SYSTÈME ET PROCÉDÉ POUR RELIER UN RÉCIPIENT DE CONCENTRÉ À UN DISPOSITIF DE TRAITEMENT DU SANG

(43) Date of publication of application: 16.10.2024
(73) Proprietor: Fresenius Medical Care Deutschland GmbH, 61352 Bad Homburg (DE)
(72) Inventor: WOHLFEIL, Florian, 61352 Bad Homburg (DE); STERZER, Rafael, 61352 Bad Homburg (DE); ROSENBERRY, Hendrik, 61352 Bad Homburg (DE); LAFFAY, Philippe, 61352 Bad Homburg (DE); DUMONT-D'AYOT, Francois, 61352 Bad Homburg (DE)
(74) Representative: Lorenz Seidler Gossel Part. mbB

(56) References cited:
- EP-A1- 1 344 550
- WO-A1-2015/090563
- WO-A1-2021/180792
- WO-A1-2022/053635

## Description

The present invention refers to a container for concentrate, a system and a method for connecting a concentrate container with a blood treatment device, preferably a dialysis machine for renal replacement therapy. Further aspects of the present disclosure refer to a blood treatment device configured to be used in a method according to the present invention.

For chronic blood treatment, especially for chronic dialysis, nowadays almost exclusively bicarbonate-based dialysis solutions are used, that are generated by the blood treatment device itself / online during treatment by diluting an acidic and a basic concentrate, preferably a powder concentrate, with water. Thus, it is established practice that the basic concentrate is generated online during the treatment, i.e. that liquid basic concentrate is continuously generated during the treatment by diluting a dry concentrate/powder concentrate with water.

For this purpose, dry powder concentrate is generally provided in a container that is fluidic ally coupled to the blood treatment device so that water can be dispensed into the container by the blood treatment device for dissolving the dry concentrate. Simultaneously the basic liquid concentrate / the bicarbonate solution generated in this way is withdrawn by the blood treatment device from the concentrate container. Such a conventional concentrate container is known from e.g. EP1344550.

According to the prior art, a certain amount of force is necessary to connect the container to the blood treatment device. In order to make the blood treatment device and the concentrate container as user-friendly as possible, conventionally the blood treatment machine and the corresponding concentrate container are configured in such a way that a connection element of the container is inserted into a connection element of the machine in a downward movement, so that gravity assists the movement of the connection element of the container into / over the connection element of the machine.

However, this configuration has the disadvantage that because of the opening of the connection element of the container being opened in a downward direction (so that it can be coupled with a corresponding connection element of the blood treatment device by moving the connection element of the container from above in a downwards movement of the connection element of the blood treatment device) after a treatment has ended and the concentrate container is removed from the blood treatment device, liquid concentrate can drip from the connection element of the container. This liquid concentrate can then collect in the connection element of the blood treatment device causing undesired crystallization and thus potential clogging of the connection element of the blood treatment device.

In addition to that, in blood treatment devices according to the prior art, liquid concentrate can spill, leak or drop from a withdrawal line of the blood treatment device configured to withdraw concentrate from the container upon disconnection of the container from the blood treatment device. Since the liquid concentrate contains solutes, crystallization may occur as an additional disadvantage to these spillages generally not being hygienic.

This liquid concentrate can then collect in the connection element of the blood treatment device causing undesired crystallization and thus potential clogging of the connection element of the blood treatment device.

Further, WO 2021/180792 A1 discloses a system and method for connecting a concentrate container with a blood treatment device without leakages. EP 1 344 550 A1 discloses a connector, container with such a connector and fluid preparation device with a mating connector for such a container. From WO 2022/053635 A1, a closing element for a fluid line is known. Finally, WO 2015/090563 A1 discloses a container. Against this background it is an object of the present invention to alleviate or even completely abolish the disadvantages of the prior art. In particular, it is an object of the present invention to provide a container that is easy to handle and connect to a blood treatment device, economically advantageous and hygienic in use.

This object is solved by the present invention, namely by a container, a system and a method comprising the features recited in the independent claims.

A container for concentrate according to the present invention, comprises:
- a hollow body for containing concentrate,
- a connection portion having at least one container connection element configured to interact with a corresponding machine connection element present on a blood treatment device to fluidically couple an inner volume of the hollow body to the blood treatment device,
- two container attachment elements arranged spaced apart from each other and configured to reversibly attach the container to two corresponding machine attachment elements present on a blood treatment device, wherein
- at least one of the two container attachment elements comprises a rounded and / or curved rotation surface configured to be placed onto a corresponding machine attachment element of the blood treatment device in an attachment position to be held there by gravity and configured to bulge in the direction of gravity in the attachment position.

The hollow body can have any desirable configuration suitable for containing a concentrate and can be a bag, cassette, cartouche, pouch or box. The hollow body can be formed of a flexible or rigid polymer material. The connection portion is preferably made of a rigid polymer material to ensure sufficient mechanical stability for connecting the container to a blood treatment device.

The rotation surface preferably is a surface configured to be placed onto a surface of a machine attachment element and configured to allow rotation of the connection portion of the container in the direction in that the container is attached to the blood treatment device. For example, the machine attachment element comprises a protruding pin and the container is attached to the blood treatment device by placing the rounded rotation surface of the container attachment element onto the protruding pin (also referred to as a stud). The container in this position orients itself and in particular its hollow body in a vertical direction, because the weight of the concentrate in the hollow body pulls downwards under the influence of gravity and the rotation surface moving on the protruding pin allows the connection portion to reflect the movement of the hollow body.

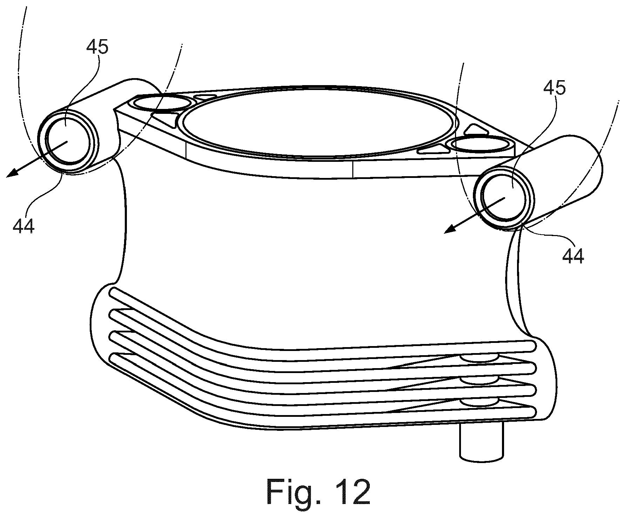

The rotation surface thus is preferably configured so that the circumference of a curvature defined by the rotation surface extends in the direction in that the container is moved to be attached to the blood treatment device. The rotation surface thus is preferably not configured so that the curvature defined by the rotation surface extends at an angle or at right angles to the direction in that the container is moved to be attached to the blood treatment device. Thus, rounded surfaces, as indicated for example by reference numeral 44 in Fig. 12 are not regarded as curved and / or rounded rotation surfaces as recited in the present disclosure.

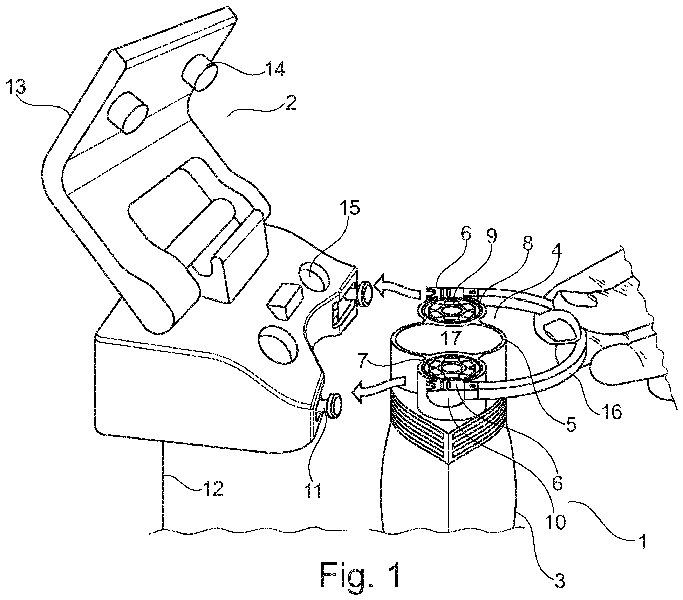

Preferably, the rotation surface is configured to allow a rocking motion, preferably back and forth along the direction of attaching the container to the blood treatment device (as indicated e.g. by the arrows in Fig. 1), when the container attachment elements are placed with their rotation surface onto the corresponding machine attachment elements. A rotation surface as recited in the present disclosure thus preferably does not mean any curved or rounded surface capable of allowing any rotation, such as e.g. a rounded surface of a plug or socket of a plug connection, but preferably indicates a surface configured to allow a container attached to a blood treatment device by its attachment elements to adopt a vertical orientation on its own accord (e.g. without any human interaction) by rotating along the rotation surface under the influence of gravity.

The shape of the rotation surface can be chosen at will, as long as preferably a rounded and / or curved surface results that allows the connection portion of the container to rock back and forth with the rotation surface of the at least one container attachment element being placed on the at least one corresponding machine attachment element.

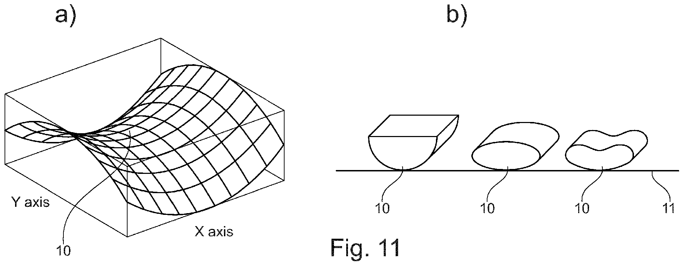

For example, the rounded and / or curved rotation surface can have a saddle shape or be formed as a half- cylinder or can have a shape between these two geometries. The ground surface of the half cylinder can be a circle, an oval or any other desirable shape, for example, a bean-shaped surface or a kidney-shaped surface, as illustrated for example in Fig. 11.

The terms "rounded" and "curved" can be understood to mean any surface at least partially comprising a continuous curvature.

According to an embodiment, both of the two container attachment elements comprise a rounded and / or curved rotation surface. The two container attachment elements can be arranged at two opposing sides of the connection portion of the container.

At least one container attachment element can comprise one or more protrusions present at an end side of the rounded and / or curved rotation surface. When viewed in the attachment direction of a user attaching the container at a blood treatment device, the protrusion(s) are preferably arranged at a distal side of the rounded and / or curved rotation surface.

The one or more protrusions can project in a first direction and the container connection element can project in a second direction that is oriented at an angle, preferably essentially at right angles, to the first direction.

The one or more protrusions can also be configured to project essentially at right angles to the direction of gravity in a position in that the container attachment elements are attached to corresponding machine attachment elements.

Alternatively or additionally, the one or more protrusions project along a curved, preferably circular, circumference defined by the rounded and / or curved rotation surface.

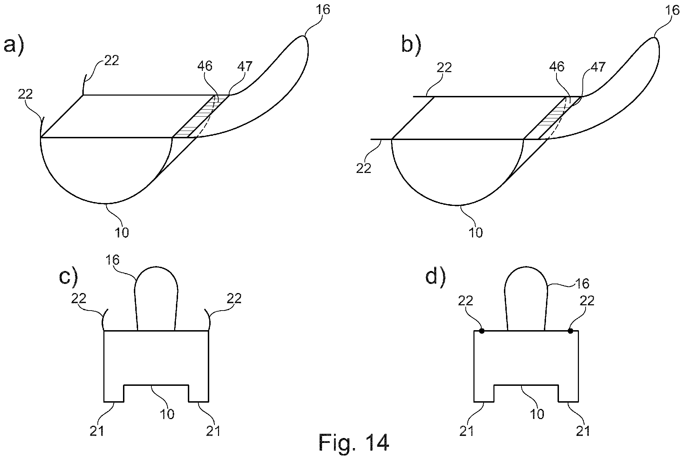

Exemplary arrangements of the protrusions relative to the rotation surface can be seen in Figs. 14 a) and 14b).

The protrusion(s) can serve the purpose of securing the container in the attachment position against an undesirable upwards movement in the vertical direction, e.g. upon a retraction of a machine connection element from the container connection element.

At the rounded and / or curved rotation surface of the container attachment element at least one rib can be present to prevent lateral movement of the container in the attachment position. The at least one rib preferably is arranged adjacent to the rounded and / or curved rotation surface and / or extends along the rounded and / or curved rotation surface.

At the rounded and / or curved rotation surface of the container attachment element two ribs can present that define a groove between them, wherein the groove is configured to at least partially receive one of the two corresponding machine attachment elements of the blood treatment device in the attachment position. The two ribs are preferably arranged at two opposing sides if the rotation surface.

When viewed in the attachment direction of a user attaching the container at a blood treatment device, the protrusion(s) are preferably arranged at a distal side of the rounded and / or curved rotation surface and a gripping portion at the user holds the container is preferably arranged at a proximal side of the rounded and / or curved rotation surface.

A linking portion can be present between the gripping portion and the rounded and / or curved rotation surface, so that a point linking the gripping portion to the container is offset from a center of gravity of the container.

When viewed against the attachment direction of a user attaching the container at a blood treatment device, the ribs are preferably arranged at the right and left sides of the rounded and / or curved rotation surface. This arrangement is illustrated e.g. in Fig. 14c) and 14d).

The groove between the two ribs can be solely defined by the two ribs and the rounded and / or curved rotation surface forming a bottom surface of the groove.

A longitudinal axis of the at least one container connection element can be oriented essentially in parallel to the longitudinal axis of the hollow body of the container. The at least one container connection element can have the form of a hollow cylinder opened at an end face of the connection portion, wherein a leading edge of the at least one container connection element comprises preferably a chamfered portion.

The at least one container connection element can comprise an outer lumen and an inner lumen preferably arranged concentrically to the outer lumen, wherein an outer surface of a wall defining the inner lumen has a polygonal, preferably a hexagonal, geometry. Preferably, the leading edge of the wall defining the inner lumen comprises a chamfered portion.

On the wall defining the inner lumen of the at least one connection element a closing element, preferably a cap, can be arranged, wherein an inner contour of the closing element arranged on the wall defining the inner lumen matches the polygonal, preferably hexagonal, geometry of the outer surface of the wall defining the inner tube.

The hexagonal configuration of the wall defining the inner lumen and the corresponding hexagonal configuration of the cap allows for a precise relative positioning of the cap and inner lumen. Preferably, the cap comprises six plates arranged adjacent to each other separated by intended breaking points and each plate is arranged on one edge of the hexagonal form of the wall defining the inner lumen. If pressure is exerted onto the plates, e.g. by a machine connection element, each plate pivots around the corresponding edge of the wall defining the inner lumen.

In order to facilitate the insertion of the machine connection element into the container connection element, the at least one container connection element can comprise at least one positioning element, preferably formed as a tooth in a leading edge of a wall defining the outer lumen of the container connection element.

The connection portion of the container can comprise a central opening for loading the hollow body with concentrate extending for a first length in a first direction and for a second length in a second direction, wherein the second length is smaller than the first length. For example, the central opening can have an oval, ellipsoid, super-elliptic, rectangular or figure-of-eight-shape or modifications of these shape, such as a rectangle with rounded corners.

According to an embodiment, two container connection elements and / or two container attachment elements are arranged at two opposing sides of the central opening so that the distance between the two container connection elements is reduced or minimized. In other words, the two container connection elements and / or two container attachment elements can be separated from each other over the second length rather than the first length.

To achieve a compact design of the connection portion of the container, in an area of juxtaposition of at least one container connection element and the central opening, the central opening can comprise a constriction portion convexly projecting into the opening of the central opening.

The constriction portion can comprise a concave section in that the connection element, preferably the outer lumen thereof, is arranged. Alternatively or additionally, a wall defining the central opening in the constriction portion at least partially forms a wall defining an outer lumen of at least one container connection element.

Another aspect of the present disclosure pertains to a blood treatment device configured to be used with a container according to the present disclosure, comprising
a housing,
two machine attachment elements stationarily arranged at the housing to that two corresponding container attachment elements of a container for concentrate are attachable, wherein at least one of the machine attachment elements comprises a holding element onto that a rounded rotation surface of a connector attachment element is to be placed in the attachment position of the container at the blood treatment device in that the container is held at the blood treatment device by gravity; and
two machine connection elements pivotably arranged at a flap pivotably arranged at the housing and each fludically linked to a fluid path arranged in the flap, wherein the two machine connection elements are configured to fluidically couple the blood treatment device to the container for concentrate by each being inserted into a corresponding container connection element, wherein the two machine connection elements are rotationally movable in the same direction to be inserted into the corresponding container connection elements upon a pivoting movement of the flap.

At least one machine connection element can be movable along a first path into a treatment position to insert the at least one machine connection element into a corresponding container connection element and at the at least one machine connection element can be movable along a second path into a rinsing position to insert the at least one machine connection element into a corresponding rinsing element of the blood treatment device.

A blood treatment device according to the present disclosure can comprise a sensor configured to detect if a container has been attached to the blood treatment device in the attachment position and a control unit configured to move the at least one machine connection element into the treatment position if a container in the attachment position has been detected by the sensor.

The control unit can be a processor, such as a digital signal processor (DSPs), general purpose microprocessor, application specific integrated circuit (ASICs), field programmable logic array (FPGAs), or other equivalent integrated or discrete logic circuitry. Accordingly, the "control unit" as used herein may refer to any of the foregoing structure or any other physical structure suitable for implementation of the described techniques. This also applies to other units, such as an evaluation unit etc.

The same holds true for the memory unit that can be any physical structure suitable for storing electronic data, such as a computer-readable medium. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer.

If in the present disclosure it is recited that a unit, such as the evaluation unit, control unit or memory unit, is configured to perform a given step, this can be understood as the unit specifically being programmed to perform the given step.

At least one machine connection element can be pivotably arranged at a lever being rotatably arranged at the housing of the blood treatment device.

At least one machine connection element can comprise a wall with a serrated leading edge configured to open a closing element, preferably a cap, arranged on a wall defining an inner lumen of a container connection element by breaking intended breaking points present in the closing element.

The serrated leading edge can comprise one or more teeth, wherein in case of multiple teeth, each tooth preferably is arranged at the wall of the at least one machine connection element such that each tooth selectively exerts pressure onto a plate of the closing element upon insertion of the machine connection element into the container connection element, wherein the closing element can comprise multiple plates separated from each other via intended breaking points.

The two machine attachment elements can each comprise a holding element comprising a protrusion, preferably formed as a stud, and / or a retaining element, preferably formed as a ridge or rip, arranged at a distance from the protrusion and configured to retain a container attached to the blood treatment device in the attachment position against a vertically upward movement, in particular when the machine connection element is retracted from the container connection element.

A blood treatment device according to the present disclosure can comprise at least one rinsing element for receiving the at least one machine connection element in the rinsing position, wherein the rinsing element preferably is formed as a trough arranged inclined at an angle to a vertical direction, preferably at an angle of inclination of 40° to 50°, wherein preferably a fluid line is connected to the rinsing element in a lower most section of the rinsing element in the vertical direction.

The fluid line can be connected to the rinsing element in a peripheral area of a bottom surface of the rinsing element and / or can project at an angle of inclination of 40° to 50° relative to the bottom surface of the rinsing element.

The at least one rinsing element can be arranged stationary relative to a housing of the blood treatment device.

In other words, the present disclosure pertains to a blood treatment device configured to be used with a container according to the present invention, comprising
- a housing,
- two machine attachment elements to that two corresponding container attachment elements of a container for concentrate are attachable, wherein at least one of the machine attachment elements comprises a holding element, e.g. in the shape of a pin or stud, onto that a rounded and / or curved rotation surface of a connector attachment element is to be placed in the attachment position of the container at the blood treatment device in that the container is held at the blood treatment device by gravity; and
- at least one machine connection element configured to fluidically couple the blood treatment device to the container for concentrate by interacting with a corresponding container connection element.

The at least one machine connection element is preferably movable along a first path into a treatment position to insert the at least one machine connection element into a corresponding container connection element and thereby fluidically couple the blood treatment device to the container.

Further, the at least one machine connection element is preferably movable along a second path into a rinsing position to insert the at least one machine connection element into a corresponding rinsing element of the blood treatment device.

The blood treatment device can comprise a sensor configured to detect if a container has been attached to the blood treatment device in the attachment position.

The sensor can be e.g. an optical sensor configured to inspect an area in that at least one container attachment element and / or at least one container connection element is arranged if a container is present in the attachment position.

Alternatively or additionally, the blood treatment device can be equipped with a mechanical sensor indicating the presence or absence of a container at the blood treatment device. For example, the mechanical sensor can have the form of a lever changing its relative position to the housing of the blood treatment device, e.g. by being depressed, when a container is attached to the blood treatment device. Thus, the relative position of the level indicates the presence or absence and / or correct attachment of the container to the blood treatment device.

The blood treatment device can further comprise a control unit configured to move the at least one machine connection element into the treatment position if a container in the attachment position has been detected by the sensor.

For this purpose, the control unit preferably is configured to operate an actuator such as a solenoid to move, e.g. push or pull, the connection element against the biasing force of a spring from the rinsing position into the treatment position.

In order to be movable between the treatment position and the rinsing position, the at least one machine connection element is preferably pivotably arranged at a lever being rotatably arranged at the housing of blood treatment device.

The at least one machine connection element can be pivotably arranged at a flap being rotatably arranged at the housing of blood treatment device. The flap is preferably movable by hand.

For example, the machine connection element can be moved in a rotatory path by moving the flap relative to the housing of the blood treatment device to juxtapose the machine connection element to the container connection element and then the machine connection element can be moved in a translational path to insert the connection element into the corresponding container connection element.

In order to reduce the peak amount of force required for opening a closing element closing the inner lumen of the container connection element, the at least one machine connection element can comprise a wall with a serrated leading edge configured to open a closing element, preferably a cap, arranged on a wall defining an inner lumen of a container connection element by breaking intended breaking points present in the closing element. The serrated leading edge can achieve a particularly targeted exertion of force onto the individual plates of the closing element.

The serrated leading edge can comprise one or more teeth, wherein in case of multiple teeth, each tooth preferably is arranged at the wall of the at least one machine connection element such that each tooth selectively exerts pressure onto a plate comprised by the closing element upon insertion of the machine connection element into the container connection element, wherein the closing element comprises multiple plates separated from each other via intended breaking points.

For example, the closing element comprises six plates and the serrated leading edge comprises three teeth that exert pressure onto three of the six plates.

The two machine attachment elements can each comprise a protrusion, preferably formed as a stud or pin, and a retaining element, preferably formed as a ridge or rip, arranged at a distance from the protrusion and configured to retain a container attached to the blood treatment device in the attachment position against a vertically upward movement, in particular when the machine connection elements are retracted from the container connection elements.

For example, if the container attachment elements are arranged on the machine attachment elements so that the rounded and / or curved rotation surfaces of the container attachment elements rest or sit on the studs or pins and the machine connection element inserted into the container connections elements is retracted upwards in vertical direction, friction may cause the container to follow the upward movement of the machine connection element. In this case, the retaining element limits the upward movement of the container to a position in that the container attachment element abuts the retaining element.

A blood treatment device according to the disclosure can further comprise at least one rinsing element for receiving the at least one machine connection element in the rinsing position, wherein the rinsing element preferably is formed as a trough that is arranged inclined at an angle to a vertical direction, preferably at an angle of inclination of 40° to 50°, wherein preferably a fluid line is connected to the rinsing element in a lower most section of the rinsing element in the vertical direction.

The inclined arrangement of the rinsing element relative to the vertical position can prevent encrustations forming in the rinsing element.

The fluid line can be connected to the rinsing element in a peripheral area of a bottom surface of the rinsing element and / or can project at an angle of inclination of 40° to 50° relative to the bottom surface of the rinsing element.

The inclined arrangement of the fluid line relative to the rinsing element relative can generate flow conditions in the rinsing element favorable for efficient rinsing.

The at least one rinsing element can be arranged stationary relative to a housing of the blood treatment device.

Another aspect of the present invention relates to a system comprising at least one container according to the present invention and at least one blood treatment device according to the present disclosure, wherein preferably the container is reversibly attached by gravity to the blood treatment device in the attachment position by means of the two container attachment elements interacting with the two machine attachment elements and / or the at least one machine connection element is fluidically coupled to the at least one container connection element.

In a system according to the present invention, preferably
- at least one container connection element comprises a wall defining an inner lumen, the wall having an outer surface with a hexagonal geometry, wherein a closing element is arranged on the wall defining the inner lumen and an inner contour of the closing element matches the hexagonal geometry of the outer surface of the wall defining the inner lumen of the container connection element, wherein the closing element comprises a closing portion comprising a plurality of plates arranged juxtaposed to each other separated by a plurality of intended breaking points; and
- at least one machine connection element comprises a wall with a serrated leading edge comprising a plurality of teeth, wherein
- the wall of the machine connection element and the closing element arranged on the wall defining the inner lumen of the container connection element are arranged in such a way that upon connection of the machine connection element with the container connection element, the teeth each exert pressure on one plate of the closing element so that each plate pivots around an edge of the hexagonal outer surface of the wall defining the inner lumen, thereby breaking at least one intended breaking point of the closing element and fluidically opening the closing element.

Yet another aspect of the present invention relates to a method for connecting a container for concentrate, preferably a container according to the present invention, to a blood treatment device, preferably a blood treatment device according to the present disclosure, comprising the step:
- reversibly attaching the container to the blood treatment device by affixing two container attachment elements to two corresponding machine attachment elements by a combination of a rotational movement and an axial movement of the container and / or the two container attachment elements relative to the blood treatment device and / or the two machine attachment elements, wherein the rotational movement and the axial movement are performed at least partially simultaneously.

In other words, embodiments of the present invention can be described as follows:
A container for concentrate according to the present invention comprises a hollow body for containing concentrate, a connection portion having an end surface and configured to connect the container to a blood treatment device,
two attachment elements preferably arranged at two opposing sides of the connection portion and configured to reversibly attach the container to two corresponding attachment elements present on a blood treatment device, and
two connection elements each comprising an opening at the flat end surface of the connection portion and configured to interact with two corresponding connection elements present on a blood treatment device to fluidically couple the container with the blood treatment device by insertion of the connection elements present on the blood treatment device into the connection elements of the container, wherein
the two attachment elements of the container each comprise a rounded rotation surface bulging in a longitudinal direction of the connection elements and the two rounded rotation surfaces are configured to each be placed onto a corresponding attachment element of the blood treatment device to be held there by gravity.

The rounded rotation surface facilitates the attachment of the container to a blood treatment device and thus makes the container more user-friendly. For example, the rounded rotation surfaces of the two attachment elements of the container can be placed onto two attachment elements of the blood treatment device that preferably have the form of studs. In this position, the weight of the container rests on the attachment elements of the blood treatment device and thus the burden on the user handling the container is reduced.

Once the rounded rotation surfaces of the two attachment elements of the container are placed onto the two attachment elements of the blood treatment device the container can be rotated, inclined or rocked on the rounded rotation surfaces, e.g. to lock the protrusions of the attachment elements of the container to retaining elements, e.g. in the form of ridges, of the attachment elements of the blood treatment device, for example by a form-fit mechanism.

Thus, the rounded rotation surfaces can offer the advantage of reducing the weight to be carried by the user while ensuring flexibility and mobility in the positioning of the container relative to the blood treatment device and thus allowing for an easy attachment of the container to the blood treatment device. Furthermore, the rounded rotation surfaces allow the container to orient itself in an upright position on its own accord due to the hollow body of the container pulling downwards under the influence of gravity.

Furthermore, the present invention preferably uses the container to collect any drops dripping from the connection elements of the blood treatment device.

For this purpose, the opening element(s) of a container according to the present invention are preferably opened in an upward direction so that the connection element(s) of the blood treatment device can be inserted into the openings / connection element(s) of the container from above in a downward motion or the connection elements of the container can be moved in an upward motion so that the connection element(s) of the blood treatment device can be inserted into the openings / connection element(s) of the container. Thus, a container according to the present invention is hygienic in use.

In addition to that, the downward movement of the connection element(s) of the blood treatment device is more user-friendly, in particular if the connection element(s) of the blood treatment device are arranged on a flap movable to a housing of the blood treatment device. As the user closes flap of the blood treatment device, the connection element(s) of the blood treatment device are preferably inserted into the connection element(s) of the container.

The two attachment elements of the container can each comprise one or more protrusions present at an end face of the rounded rotation surface, wherein the one or more protrusions project in a first direction and the two connection elements project in a second direction that is preferably oriented essentially at right angles to the first direction, or the one or more protrusions project along a preferably circular path or circumference defined by the rounded rotation surface.

This is illustrated under reference to Figs. 14a) to 14d) and explained in more detail in the discussion of these figures. For example, a curved and / or rounded rotation surface is formed by the bulging portion of a half cylinder. The half-cylinder comprising the rounded rotation surface has four sides.

At two opposing sides of the rotation surface two ribs can be arranged to prevent a lateral movement of the container when the rounded rotation surface sits on a machine attachment element by gravity. The two ribs preferably define a groove between them with the bottom surface of the groove being defined by the rounded rotation surface.

At two opposing sides of the rotation surface, in particular at the two opposing sides comprising the two ribs, two protrusions can be present. The protrusions can project along a circumference defined by the curvature of the rounded rotation surface. Alternatively, the protrusions can project along a plane defined by the flat surface of the half cylinder. In this arrangement the protrusions preferably extend at right angles to the connection element(s) of the container.

A gripping portion is preferably arranged at a side of the rounded rotation surface that is opposed to the side of the rounded rotation surface comprising the two protrusions.

Preferably, when viewed in a direction of attachment of the container at a blood treatment device by a user, the protrusions are arranged at a distal end of the rounded rotation surface and the gripping portion is arranged at a proximal end of the rounded rotation surface.

The gripping portion is preferably linked to the rounded rotation surface by a linking portion so that a connection point of the gripping portion to the linking portion does not coincide with a center of gravity of the connection portion of the container. This has the effect that if a user grips the container at the gripping portion, the connection portion of the container tilts out of a vertical position into an inclined position. This can facilitate to attachment of the container to the blood treatment device.

The one or more protrusions preferably serve to reversibly lock the attachment elements of the container to the attachment elements of the blood treatment device by a form-fit mechanism. For example, the one or more protrusions can be inserted into a recess comprised by an attachment element of the blood treatment device. Alternatively, the one or more protrusions can interact with a protrusion, e.g. a rip or ridge, of the blood treatment device forming a retaining element that limits the movement of the container attached to the blood treatment device.

Furthermore, the one or more protrusions can interact with a retaining element of the blood treatment device, e.g. a rib or ridge, to retain the container in position attached to the blood treatment device when the connection elements of the blood treatment device are removed upwards in a vertical direction from the connection elements of the container.

The one or more protrusions can be arranged at two opposing sides of the rounded rotation surface. The one or more protrusions can be arranged at a first side of the rounded rotation surface and a handle for gripping the container can be arranged on a second side of the rounded rotation surface that is preferably arranged opposite to the first side.

At two opposing sides of each rounded rotation surface of each attachment element of the container, two ribs can be present that extend along the length of the rounded rotation surface and define a groove between them, wherein the groove is configured to at least partially receive a corresponding attachment element of the blood treatment.

In other words, the ribs and the groove can define a poulie-like structure configured to run on an attachment element of the blood treatment device that preferably has the form of a stud. The two ribs stabilize the attachment element of the container in a lateral direction on the attachment element of the blood treatment device that is at least partially inserted into the groove. In principle, only one rib on one side of the rounded rotation surface can suffice to limit lateral movement.

The groove between the two ribs can be defined solely by the two ribs and the rounded rotation surface forming a bottom surface of the groove. In other words, the two ribs and the rounded rotation surface can form the groove for the insertion of the attachment element of the blood treatment device.

According to an embodiment, a longitudinal axis of the two connection elements of the container is oriented essentially in parallel to the longitudinal axis of the hollow body of the container and the two connection elements each can comprise a hollow cylinder opened at the flat end surface of the connection portion, wherein a leading edge of each connection element comprises a chamfered portion.

The chamfered portion facilitates the insertion of the connection elements of the blood treatment machine into the connection elements of the container.

Each connection element can comprise an outer tube and an inner tube preferably arranged concentrically to the outer tube, wherein an outer surface or circumference of the inner tube preferably has a polygonal, preferably a hexagonal, geometry. The inner tube can be regarded as a hollow cylinder and is preferably configured to conduct fluid in and out of the hollow body of the container. The inner surface or circumference of the inner tube that defines the lumen of the inner tube can have any geometry and preferably has a circular geometry.

The hexagonal geometry of the outer surface or circumference of the inner tube is advantageous because it interacts with a closing element or cap closing the lumen of the inner tube, in order to reduce the force required to open the closing element or cap.

For example, on the inner tube of one or each connection element of the container a closing element, preferably a cap, can be arranged wherein an inner contour of the closing element arranged on the inner tube matches the polygonal, preferably hexagonal, geometry of the outer surface of the inner tube. This ensures an exact positioning of the closing element on the inner tube as in contrast to e.g. a circular contour of the inner tube and closing element, the closing element can not rotate on the inner tube.

The exact positioning of the closing element on the inner tube can have the advantage that individual plates of the closing element can be arranged in such a position that if a machine connection element is inserted into the container connection element, individual teeth of the serrated leading edge of the machine connection elements can exert pressure onto a desired position (e.g. right in the middle) of a plate of the closing element.

For example, the serrated leading edge can comprise three teeth that simultaneously hit the middle of three corresponding plates of the closing element upon insertion of the machine connection element into the container connection element. This way the maximum force required for opening the closing element can be reduced.

Preferably, the closing element or cap comprises a closing portion formed by a plurality of plates separated from each other by intended breaking points, e.g. local areas of selective material thinning.

Upon insertion of a connection element of the blood treatment device into a connection element of the container, the connection element of the blood treatment device exerts pressure onto at least one plate of the closing portion of the closing element in such a way that the at least one plate rotates around an edge or side of the hexagonal contour of the outer surface of the inner tube whereby at least one intended breaking point is broken and the closing element is fluidically opened.

In order to ensure a correct mounting of the container to the blood treatment device, at least one connection element can comprise at least one positioning element, preferably formed as a tooth in a leading edge of an outer tube of the connection element projection from an end surface of the connection portion of the container, configured to interact with a corresponding connection element of a blood treatment device to ensure a defined mounting position of the container at the blood treatment device.

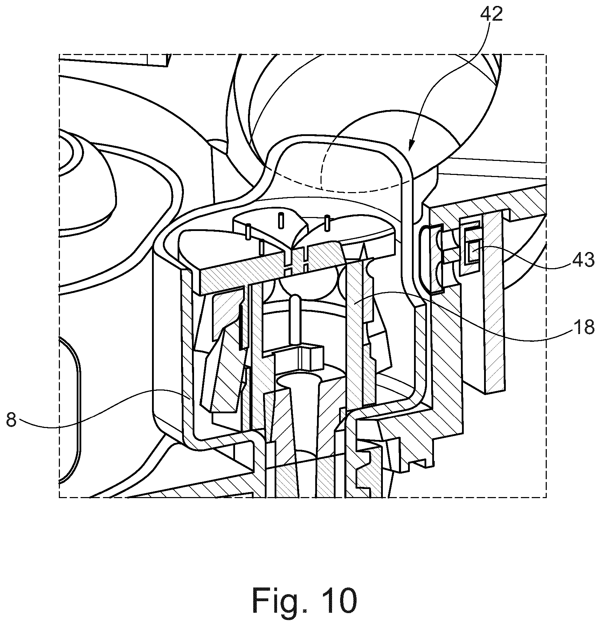

The positioning element can also serve the purpose of guiding the connection element of the blood treatment device into a connection element of the container. An example of such a positioning element can be seen in Fig. 10.

In order to achieve a compact design of the connection portion of a container according to the present invention while ensuring sufficient space for the connection elements of the container, the shape of a central opening in the connection portion can be optimized. The central opening serves the purpose of filling the container e.g. with dry or liquid concentrate.

For example, the connection portion can comprise a central opening preferably of an oval or approximately oval shape, ellipsoid or other shape comprising a first length and a second length wherein the second length is shorter or smaller than the first length and the two connection elements are preferably arranged at two opposing sides of the central opening so that the distance between the two connection elements is reduced or minimized. In other words, the two connection elements are preferably arranged at the two opposing sides of the oval or approximately oval central opening that are closest to each other.

In an area of juxtaposition of at least one connection element and the central opening, the central opening can comprise a constriction portion convexly projecting into the opening of the central opening. In this embodiment, the shape of the central opening can be regarded as a an hourglass shape with a rather modest constriction, wherein the outer tubes of the connection elements of the container are nestled snuggly on both sides into the curves of the constriction of the central opening.

In other words, the constriction portion can comprise a concave section in that the connection element, preferably an outer tube thereof, is arranged.

This arrangement allows the connection elements of the container to be arranged more closely together so that larger diameters of the outer tubes of the connection elements can be realized without increasing the overall dimensions of the connection portion of the container.

Connection elements with relatively large outer tubes are advantageous, because they allow for closing elements / caps with larger plates that require less force to open, due to improved leverage of the machine connection element exerting force on plates of larger diameter caps.

Another aspect of the disclosure relates to a blood treatment device, preferably a dialysis device, configured to be used with a container according to the present invention.

For example, the container can comprise concentrate that is used by the blood treatment device to generate a dialysis solution or dialysate. For this purpose, the connection element(s) of the blood treatment device can be fluidically coupled to the connection element(s) of the container.

Preferably, each connection element of the blood treatment device comprises a inner hollow tube or inner lumen for conducting fluid and an outer hollow tube for opening a closing element present on an inner lumen or inner hollow tube of a corresponding container connection element. The outer hollow tube of the machine connection element is thus preferably not configured to conduct fluid.

Each connection element of the container comprises an inner lumen or inner hollow tube for conducting fluid in and out of the container and an outer lumen or outer hollow tube surrounding the inner lumen or inner hollow tube for protecting inner lumen or inner hollow tube and protecting a closing element arranged on the inner lumen or inner hollow tube.

Preferably, one container connection element is used to conduct fluid into the hollow body of the container and the other container connection element is used to conduct fluid out of the hollow body of the container.

The closing element closes the inner lumen or inner hollow tube of the container connection element and thereby guards the fluid path of the inner lumen or inner hollow tube from contamination.

Upon connection of a machine connection element to a container connection element, the outer hollow tube of the machine connection element exerts pressure onto the closing element present on the inner hollow tube of the container connection element until the closing element breaks and is opened.

After the closing element has been opened, the inner hollow tube of the machine connection element is inserted into the inner hollow tube of the container connection element, so that the blood treatment device is fluidically connected to the container. Alternatively, the inner hollow tube of the container connection element is inserted into the inner hollow tube of the machine connection element, so that the blood treatment device is fluidically connected to the container.

The outer diameter of the inner hollow tube of the container connection element and the inner diameter of the inner hollow tube of the machine connection element, or vice versa, are chosen in such a way that a fluid-tight connection between the blood treatment device and the container is achieved.

A blood treatment device according to the disclosure can comprise a housing,
two attachment elements to that corresponding attachment elements of a container for concentrate can be attached, wherein the two attachment elements project in a direction projecting essentially at right angles from a surface of the housing, and
two connection elements configured to fluidically couple the blood treatment device to a container for concentrate, wherein
the two connection elements are preferably movable along a first path to insert the two connection elements into two corresponding connection elements of a container preferably by a combined rotatory and translational movement and the two connection elements are movable along a second path to insert the two connection elements into two corresponding rinsing elements of the blood treatment device preferably by a rotatory movement.

The two attachment elements can project from a surface of the housing of the blood treatment device in a direction projecting essentially at right angles to the direction of gravity.

In the following the arrangement of two connection elements of the blood treatment device (also referred to as machine connection elements) is described. Of course, this disclosure also pertains to embodiments in that only one machine connection element is present.

For example, the two connection elements of the of the blood treatment device can be arranged on a flap pivotably attached at the housing of the blood treatment device. The flap (and thus the connection elements arranged thereon) can rotate around a pivoting point in that the flap is attached to the housing of the blood treatment device. Further, the two connection elements of the blood treatment device themselves can be pivotably arranged on the flap. The two connection elements can thus rotate around a pivoting point in that the connection elements are arranged at the flap.

The flexibility of the movement of the connection elements of the blood treatment device allows for a stationary arrangement of the rinsing elements at the housing of the blood treatment device.

As the connection elements of the blood treatment device can be moved to be inserted into the corresponding container connection elements if the blood treatment device is to be operated for treatment and the connection elements of the blood treatment device can be moved to be inserted into the rising elements of the blood treatment device if the blood treatment device is to be operated for treatment, it is not necessary to arrange the rinsing elements to be movable relative to the housing of the blood treatment device as is known from some prior art solutions.

For example, according to some prior art solutions, the rinsing elements are arranged movable relative to the housing of the blood treatment device and can even be configured retractable into the machine housing. This has obvious hygienic disadvantages. With a configuration according to the present invention, the improved flexibility of the movement of the connection elements of the blood treatment device obliterates the need for movable rinsing elements.

For example, according to the present invention it is not necessary any more to move the rinsing elements of the blood treatment device in order to insert the connection elements of the blood treatment device into the rinsing elements, e.g. by retracting the rinsing elements into the main body of the blood treatment device. Thus, the arrangement according to the present invention improves hygiene.

The two connection elements of the blood treatment device can be pivotably arranged at a lever rotatably arranged at the blood treatment device, so that the two connection elements are smoothly movable from a movement along a circular path to an axial movement to insert the two connection elements of the blood treatment device into two corresponding connection elements of a container, preferably by axially moving the two connection elements of the blood treatment parallel to a longitudinal direction of the housing of the blood treatment device and / or parallel to the direction of gravity.

Thus, for insertion of the two connection elements of the blood treatment device into two connection elements of the container, the two connection elements of the blood treatment device are preferably first moved along the first circular path and then moved in an axial direction for insertion into the connection elements of the container.

The blood treatment device can comprise a sensor for detecting the presence of a container attached to the blood treatment device. A control unit of the blood treatment device can be configured to set an angular position of the machine connection elements relative to the lever at that the connection elements are rotatably or pivotably arranged.

For example, if the presence of a container attached to the blood treatment device is detected by the sensor, the control unit can move the connection elements into a first angular position relative to the lever. This position can be regarded as a treatment position, because the connection elements are arranged to be inserted into corresponding container connection elements.

If the absence of a container attached to the blood treatment device is detected by the sensor, the control unit can move the connection elements into a second angular position relative to the lever. This position can be regarded as a rinsing position, because the connection elements are arranged to be inserted into corresponding rinsing elements. In the second angular position (rinsing position), an angle defined by the lever and the longitudinal axis/es of the machine connection element(s) is preferably smaller than in the first angular position (treatment position).

Further, the connection elements of the blood treatment device can be configured to open a closing element / cap present on the connection elements of the container.

For example, the two connection elements of the blood treatment device can each comprise an outer tube with a serrated leading edge configured to open a closing element, preferably a cap, arranged on an inner tube of a connection element of a container by sequentially breaking intended breaking points present in the closing element.

The length of the tooth from a tip of the tooth to its base can be e.g. between 1 mm and 3 mm, preferably 2 mm. The connection elements of the blood treatment device can each comprise an outer tube having a diameter of between 13 mm and 16 mm, preferably between 14mm and 15 mm. The connection elements of the blood treatment device can further each comprise an inner tube having a diameter of between 4 mm and 6 mm, preferably approximately 5 mm.

In the case of only one tooth, the tooth is the part of the connection element that contacts the closing element first, thereby exerting force onto the closing element, e.g. a plate thereof, in a locally focused fashion.

In case of multiple teeth of differing lengths, the teeth contact the closing element successively.

For example, a closing element can comprise six plates. In case of multiple teeth of the same length, e.g. three teeth of equal length, the peak force required for opening the closing element can be reduced. For example, the three teeth can first exert force onto three plates of the closing element.

The fact that the force is exerted into the closing element in three ideally equally spaced points prevents the closing element from undesired tilting. Then upon a further motion of the outer tube of the machine connection element the edge of the outer tube of the machine connection element exerts pressure onto the three remaining plates of the closing element.

In either case, the maximum force required for fluidically opening the closing element can be reduced.

The attachment elements of a blood treatment device according to the present invention are preferably configured to interact with the attachment elements of a container according to the present invention.

For example, the two attachment elements of the blood treatment device can each (or only one of them) comprise a protrusion, preferably formed as a stud, and a retaining element, preferably formed as a ridge or rip, arranged at a distance from the protrusion preferably upwards in the vertical direction and configured to retain a container whose attachment elements are attached to the attachment elements of the blood treatment device in position in a vertical direction, in particular when the connection elements of the blood treatment device are retracted from the connection elements of the container.

When the connection elements of the blood treatment device are inserted into the connection elements of the container, the container can be reversibly held to the connection elements of the blood treatment device due to a friction fit.

If the connection elements of the blood treatment device are then withdrawn or retracted form the connection elements of the container in an upward direction along the vertical direction, the container could in principle be moved upwards together with the connection elements of the blood treatment device.

This undesired movement of the container can be prevented by a the retaining element of the blood treatment device that limits the upward movement of the container in a position in that the container, in particular an attachment element thereof, abuts at the retaining element.

In order to further improve hygiene, the rinsing elements of a blood treatment device according to the present invention have been optimized.

For example, the two rinsing elements that preferably are formed as troughs can be arranged at the blood treatment device inclined at an angle to a vertical direction and / or a direction of gravity and / or a longitudinal direction of the housing of the blood treatment device, preferably at an angle of inclination of 40° to 50°, and a fluid line is connected to each rinsing element preferably in a lower most section of each rinsing element in the vertical direction and / or a longitudinal direction of the main body of the blood treatment device.

This has the advantage that liquid present in the rinsing elements gathers in the lower most section of each rising element where it can be removed via the fluid line. Thus, crystallization of remnants of fluid remaining in the rinsing elements can be avoided.

The fluid line can be connected to each rinsing element in a peripheral area of a bottom surface of each rinsing element and / or can project at an angle of inclination of 40° to 50° relative to the bottom surface of each rinsing element.

This has the advantage that turbulence is introduced in the fluid flowing through the rinsing elements ensuring a thorough cleaning and rinsing of the rinsing elements.

The two rinsing elements are arranged preferably stationary relative to a main body and / or side surface of the blood treatment device.

Another aspect of the present invention pertains to a system comprising at least one container according to the present invention and at least one blood treatment device according to the present disclosure, wherein preferably the container is reversibly attached to the blood treatment device by means of the two attachment elements present on the container interacting with the two attachment elements present on the blood treatment device and / or the two connection elements of the blood treatment device are fluidically coupled to the two connection elements of the container.

According to an embodiment of a system according to the present invention, at least one connection element of the container comprises an inner tube having an outer surface with a hexagonal geometry, wherein a closing element is arranged on the inner tube and an inner contour of the closing element matches the hexagonal geometry of the outer surface of the inner tube of the connection element, wherein the closing element comprises a closing portion comprising a plurality of plates arranged juxtaposed to each other separated by a plurality of intended breaking points; and at least one connection element of the blood treatment device comprises a hollow tube with a serrated leading edge comprising at least one tooth, wherein the tube of the connection element of the blood treatment device and the closing element arranged on the inner tube of the connection element of the container are arranged in such a way that upon an insertion of the connection element of the blood treatment device into the connection element of the container, the tooth exerts pressure on at least one plate (but not all the plates) of the closing element so that the at least one plate pivots around an edge of the hexagonal outer surface of the inner tube, thereby breaking at least one intended breaking point of the closing element and fluidically opening the closing element, so that the container is fluidically connected to the blood treatment device.

By this arrangement, preferably the peak amount of force required for opening the closing element can be reduced.

Another aspect of the invention pertains a method for connecting a container for concentrate, preferably a container according to the present invention, to a blood treatment device, preferably a blood treatment device according to the present disclosure, comprising the step:
- reversibly attaching the container to the blood treatment device by affixing at least one attachment element of the container to at least one attachment element of the blood treatment device by a combination of a rotational movement and an axial movement of the container and / or attachment element of the container relative to the blood treatment device and / or attachment element of the blood treatment device, wherein the rotational movement and the axial movement are performed at least partially simultaneously.

In other words, due to the unique poulie-like design of the attachment elements of the container comprising rounded rotation surfaces and / or grooves for at least partially receiving the attachment elements of the blood treatment device, the motion to be performed in order to attach the container to the blood treatment device is also unique, as it reflects the structural configuration of the container and blood treatment device.

The at least partially simultaneous rotational movement and axial movement allows for a quick and easy, and thus user-friendly, attachment of the container to the blood treatment device. The rotational movement of the connection portion of the container can be at least partially caused by gravity, as the weight of the hollow body of the containers pulls upwards thereby moving the container into an upright position.

It is to be noted that the present disclosure is not limited to the combinations of features explicitly disclosed therein.

Further features, advantages and technical effects of the present invention become apparent from the drawings showing preferred embodiments of the invention. The drawings merely serve purposes of illustrating the invention but do not purport to limit the invention, which is defined by the appended claims. In the drawings, the same or similar components are denoted by the same reference signs.

In the drawings:
Fig. 1 illustrates how a container according to an embodiment of the invention is attached to a blood treatment device according to an embodiment of the disclosure;
Fig. 2 shows a top view of a connection portion of a container according to an embodiment of the invention;
Fig. 3 shows a bottom view of the connection portion of Fig. 2;
Fig. 4 shows a top view of a connection portion of a container according to another embodiment of the invention;
Fig. 5 illustrates the attachment elements of a blood treatment device according to an embodiment of the disclosure;
Fig. 6 illustrates a state in that a container according to the present invention is attached to a blood treatment device according to an embodiment of the disclosure and the connection elements of the blood treatment device are being inserted into the connection elements of the container;
Fig. 7 a) shows a blood treatment device according to an embodiment of the disclosure in a treatment position;
Fig. 7b) shows the blood treatment device of Fig. 7a) in a rinsing position;
Fig. 8a) illustrates how the connection elements of the blood treatment device are moved between the treatment position and the rinsing position;
Fig. 8b) further illustrates how the connection elements of the blood treatment device are moved between the treatment position and the rinsing position;
Fig. 9a) shows a top view of a rinsing trough to be used with a blood treatment device according to the present disclosure;
Fig. 9b) shows a sectional view of the rinsing trough of Fig. 9a;
Fig. 10 shows an example of a positioning element of a container connection element;
Fig. 11a) illustrates a saddle-shaped rotation surface;
Fig. 11b) illustrates other possible geometrical configurations of rotation surfaces;
Fig. 12 shows an example of a connection portion of a container comprising rounded surfaces that are not rotation surfaces in the sense of the present disclosure;
Fig. 13 shows an example of an embodiment of the present invention in contrast to the connection portion shown in Fig. 12;
Fig. 14a) illustrates a schematic overview of a rotation surface adjacent to that protrusions are arranged that follow the circumference defined by the curvature of the rotation surface;
Fig. 14b) illustrates a schematic overview of a rotation surface adjacent to that protrusions are arranged that project at right angles to the direction of gravity when the rotation surface is placed onto a machine attachment element in an attachment position;
Fig. 14 c) illustrates a view of the arrangement of Fig. 14a) when viewed from the left side in Fig. 14a) in that the two ribs are visible; and
Fig. 14d) illustrates a view of the arrangement of Fig. 14b) when viewed from the left side in Fig. 14b) in that the two ribs are visible.

Fig. 1 illustrates how a container 1 according to an embodiment of the invention is attached to a blood treatment 2 device according to an embodiment of the disclosure.

The container 1 comprises a hollow body 3 in the form of a pouch for containing concentrate and a connection portion 4 having a flat end surface 5.

The container 1 comprises two attachment elements 6 arranged at two opposing sides of the connection portion 5 and two connection elements 7 arranged on opposite sides of a central opening 17 and each comprising an outer tube 8 and an inner tube that is closed by a cap 9 comprising multiple plates arranged similarly to the petals of a flower.

The two attachment elements 6 of the container 1 each comprise a rounded rotation surface 10 bulging in a longitudinal direction of the connection elements 7 and the main body 3 and configured to be placed onto a corresponding attachment element 11 of the blood treatment device 2 to be held there by gravity.

The attachment elements 11 of the blood treatment device preferably have the shape of studs.

The blood treatment device 2 comprises a housing 12 and a movable flap 13 that is pivotably arranged at the main body. The flap 14 comprises two connection elements 14 that can be inserted into the connection elements 7 (treatment position) of the container 1 or into two rinsing elements 15 of the blood treatment device (rinsing position).

As shown in Fig. 1, in order to attach the container 1 to the blood treatment device 2, a user grips the container at a gripping portion 16 and moves the container towards the blood treatment device with a combined axial and rotational movement as indicated by the arrows in Fig. 1. Gravity then causes the container to rotate into an upright position, after the attachment elements of the container have been attached to the attachment elements of the blood treatment device.

The blood treatment device can comprise a control unit and a sensor for detecting a container 1 attached to the blood treatment device and if a container has been detected, the control unit can move the flap 13 and / or connection elements 14 from the rinsing position into the treatment position.

According to an embodiment, the control unit moves the connection elements 14 from the rinsing position into the treatment position by altering an angle between the connection elements 14 and a lever to that the connection elements 14 are pivotably attached. The flap 13 is preferably moved manually.

In the following section, the connection portion of a container according to the present invention is described in more detail.

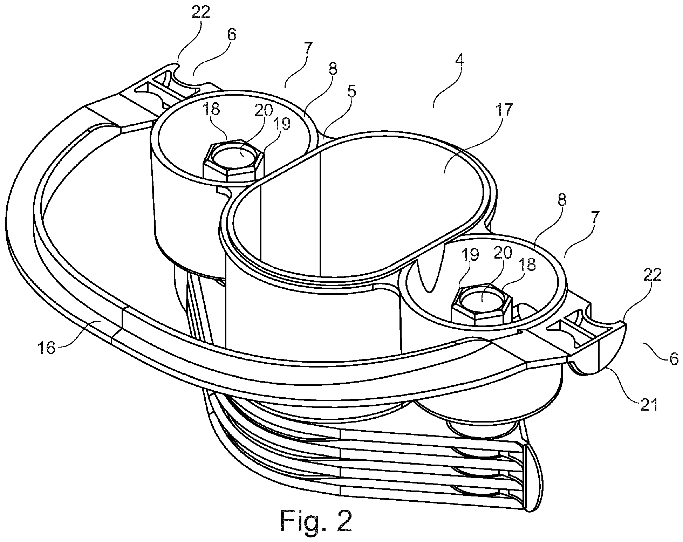

Fig. 2 shows a top view of a connection portion 4 of a container according to an embodiment of the invention.

In Fig. 2 it can be seen that the connection elements 7 of the container 1 each comprise an outer tube 8 and an inner tube 18 arranged concentrically to the outer tube 8. Each inner tube 18 comprises an outer surface or circumference 19 that has a hexagonal geometry and an inner surface or circumference 20 that has a circular geometry. In other words, the inner lumen of the inner tube 18 has a cylindrical shape. The inner lumen is configured to conduct fluid in and / or out of the container.

The central opening 17 has an approximately oval or elliptical shape and the two connection elements 7 are arranged at the two opposing sides of the central opening 17 that are closest together.

The attachment elements 6 in this embodiment comprise two ribs 21 that define a groove between into that a stud 11 of a blood treatment element can be at least partially inserted.

Each attachment element 6 comprises a protrusion 22 arranged at a side of the rotation surface opposite to the gripping portion 16. The protrusions 22 are configured to interact with two corresponding protrusions of the blood treatment device acting as retaining elements in order to retain the container 1 in position upon retraction of the connection elements 14 of the blood treatment device form the connection elements 7 of the container.

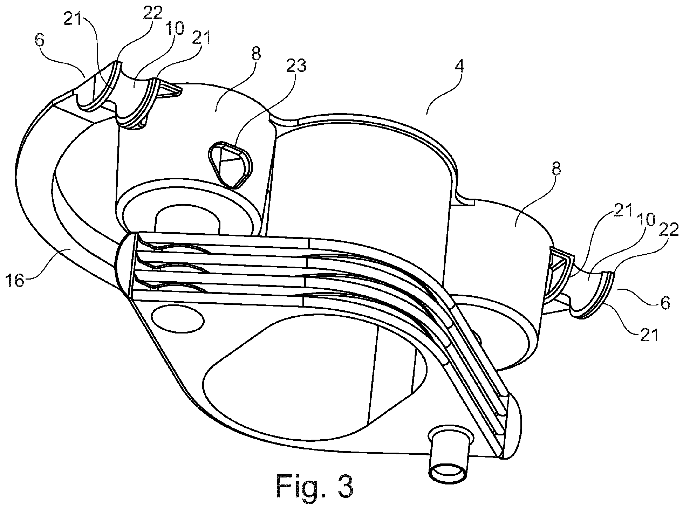

Fig. 3 shows a bottom view of the connection portion 4 of Fig. 2. In this view, the structure of the attachment elements 6 can be seen.

Each attachment element 6 comprises a rounded rotation surface 10 bulging convexly in a longitudinal direction of the connection elements 7 or outer tubes 8. On two opposing sides of each rotation surface 10 two ribs 21 are arranged that extend along the rounded rotation surface 10. The two ribs 21 and the rotation surface 10 define a groove between them, similar to a poulie.

If the attachment elements 6 are placed onto the studs 11, the studs are inserted into the groove so that each rounded rotation surface 10 rests on a stud 11. The container can in this position rotate on the rotation surface 10 and slide on the studs 11 while the ribs 21 provide lateral stabilization.

Due to the weight of the concentrate in the pouch of the container pulling down due to gravity, the container moves itself into an upright position by a rotation and / or rocking motion of the rotation surface 10 on the studs 11. The protrusions 22 rest in a position abutting the ridges 31.

In order to detach the container from the blood treatment device again, a user grips the container at the gripping portion 16, thereby causing a rotation of the container along the rotation surface 10 rolling on the stud 11 and separating the protrusions22 from the ridges 31.

Furthermore, in Fig. 3 an inspection window 23 present in at least one outer tube 8 can be seen. This inspection window 23 allows a sensor of the blood treatment device, e.g. an optical sensor, to inspect e.g. a closing element or cap present on an inner tube of 18 of the container 1 attached to the blood treatment device for integrity.

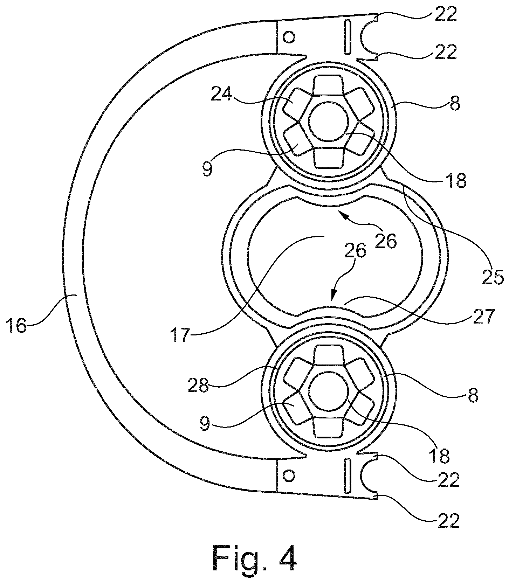

Fig. 4 shows a top view of a connection portion 4 of a container according to another embodiment of the invention. In this view, the two caps 9 used for closing the inner tube 18 of the connection elements 7 of the container 1 have opened / broken by the connection elements 14 of the blood treatment device exerting pressure onto the plates 24 of the caps 9. For this reason, in the view in Fig. 4 the inner tubes 18 are visible as opposed to the view in Fig. 1.

In the embodiment of Fig. 4 the wall 25 defining the central opening 17 has a slight hourglass contour and comprises a construction portion 26 indicated by the arrows in Fig. 4. The constriction portion 26 comprises a convex section 27 projecting into the central opening and a corresponding concave section 28 into that the outer tube 8 of the connection elements 7 are nestled.

This arrangement allows for larger diameter outer tubes 8 while keeping the dimension of the connection portion between the attachment elements 6 compact.

In the following section, selected features of a blood treatment device according to the present invention are described in more detail.

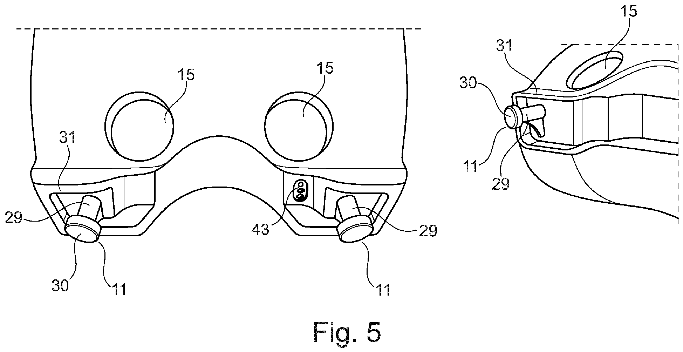

Fig. 5 illustrates the attachment elements 11 of a blood treatment device 2 according to an embodiment of the disclosure. In this embodiment, the studs comprise an elongated neck 29 that is configured to be at least partially inserted into a groove defined between the two ribs 21 of the attachment elements of a container 1, and a stopper 30 stopping the container 1 from sliding off the studs 11.

Adjacent to each stud 11 in vertical direction upwards and / or displaced from each stud in the direction of the rinsing elements 15 the blood treatment device comprises a ridge 31 configured to prevent an upward movement (as indicated by the arrows in Fig. 5) in the vertical direction of the container upon retraction of the connection elements 14 from the connection elements 7.

As the connection elements 14 are retracted upwards in vertical direction from the connection elements 7, the protrusions 22 of the attachment elements 6 of the container 1 abut the ridges 31 of the blood treatment device and thereby limit the upward movement of the container.

In the embodiment shown in Fig. 5 each ridge 31 at least partially surrounds each stud 11, for example on three sides.

The blood treatment device shown in Fig. 5 is also equipped with a sensor 43 configured to inspect a cap 6 present on the inner tube 18 through the inspection window 23.

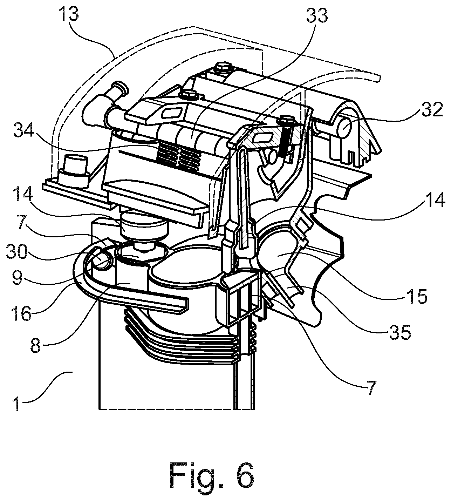

Fig. 6 illustrates a state in that a container 1 according to the present invention is attached to a blood treatment device 2 according to an embodiment of the disclosure and the connection elements 14 of the blood treatment device 2 are in the process of being inserted into the connection elements 7 of the container 1.

As can be seen in Fig. 6, the flap 13 and thus the connection elements 14 mounted thereon are arranged pivotably around a first pivot axis 32 at the blood treatment device.

Furthermore, the connection elements 14 are arranged pivotably around a second pivot axis 33 at the flap 13.

A movement around the second pivot axis 33 allows to move the connection elements from a position for insertion into the connection elements 7 of the container 1 (treatment position; shown in Fig. 6) to a position for insertion into the rinsing elements 15 (rinsing position).

The blood treatment device 2 mostly remains in the rinsing position if no treatment is performed. An actuator, e.g. a solenoid, can be provided to selectively move, e.g. push or pull, the connection elements against the biasing force of the spring from the rinsing position into the treatment position.

In Fig. 6, the arrangement of the rinsing elements 15 at the blood treatment device 2 can also be seen. The rinsing elements 15 are arranged inclined relative to a vertical direction or to a direction defined by a side surface of the blood treatment device and a fluid line 35 is connected to each rinsing element 15 in the lower most section so that fluid gathering in the rinsing elements can be removed.

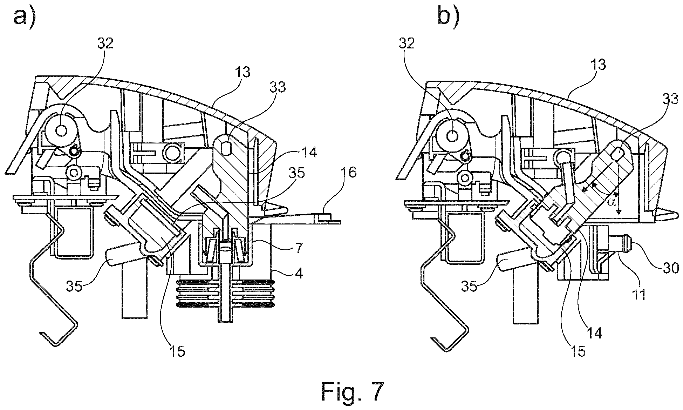

Fig. 7 a) shows a blood treatment device according to an embodiment of the disclosure in a treatment position. In this position, the flap 13 is closed, i.e. lowered towards the container 1 attached to the blood treatment device 2.

As shown in Fig. 7a, the connection elements 14 of the flap 13 are inserted into the connection elements 7 of the container 1. In this position, the rinsing elements 15 of the blood treatment device are covered or closed by a surface 36 of the flap 13. This prevents the rinsing elements 15 from being contaminated.

As shown in Fig. 7a, the connection elements 14 are arranged in this this position in an axial direction parallel to the vertical direction. In other words, the connection elements 14 in Fig. 7a project along the vertical direction downwards into the connection elements 7 of the container.

Fig. 7b) shows the blood treatment device of Fig. 7a) in a rinsing position. In this position, the connection elements 14 are rotated for an angle α shown in Fig. 7b) around the axis 33 from the treatment position shown in Fig. 7a) and are inserted in the rinsing elements 15 of the blood treatment device.

The angle α shown in Fig. 7b) corresponds in this example to 43°. In other words, in the rinsing position, the connection elements are rotated by an angle α, preferably an angle of between 40° and 50°, relative to their position in the treatment mode.

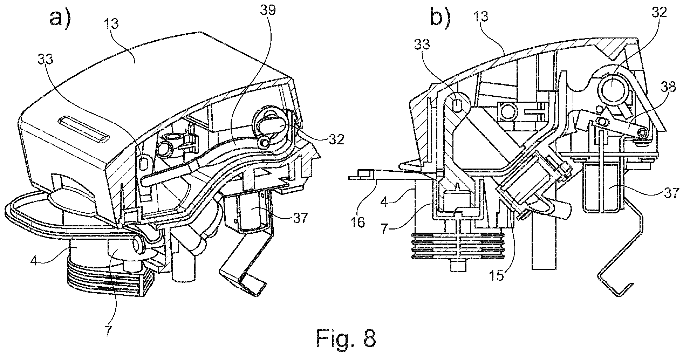

Figs. 8a) and 8b) illustrate how the connection elements 14 of the blood treatment device are moved between the treatment position and the rinsing position.

For this purpose, the blood treatment device 2 is equipped with a solenoid 37 that is configured to actuate a lever 38 that acts on the connection elements 14 via the lever 39 to push the connection elements 14 into the treatment position against the biasing force of a spring not shown in Fig. 8. The solenoid 37 is preferably activated by the control unit of the blood treatment device upon detection of a container being connected to the blood treatment device by the sensor.

If the solenoid 37 is not active, the spring pulls the connection elements 14 back into their rinsing position.

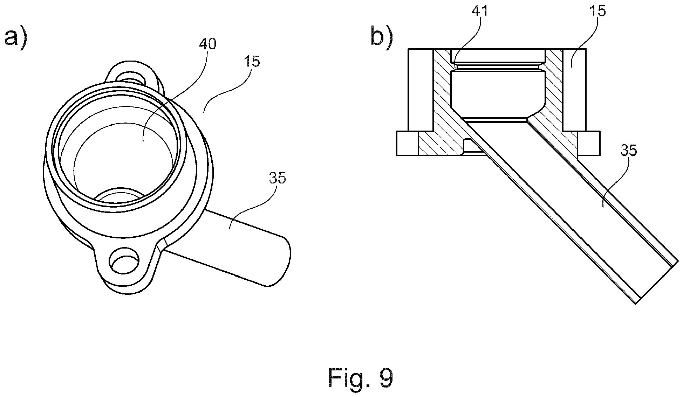

Fig. 9a) shows a top view of a rinsing element 15 or trough to be used with a blood treatment device 2 according to the present disclosure. The fluid line 25 is connected to the rinsing element 15 in a lower most section of the rinsing element 15.

In Fig. 9a, the rinsing element 15 is shown in an inclined position similar to its mounting position at a blood treatment device according to the present disclosure.

The fluid line 35 is connected to the rinsing element 15 in a peripheral section of the bottom portion 40 of the rinsing element 15. Furthermore, the fluid line 35 is arranged at an angle to a bottom surface of the rinsing element 15 to improve rinsing of the rinsing element by generating turbulences in the rinsing fluid.

Fig. 9b) shows a sectional view of the rinsing element 15 of Fig. 9a. In this view, a circular sealing element 41 running along the inner circumference of the rinsing element 15 can be seen.

This sealing element can be e.g. an O-ring and serves the purpose of sealing a connection between the connection elements 14 of the blood treatment device 2 and the rinsing elements 15 in the rising position.

As shown in Fig. 10, the outer tube 8 of a container connection element can comprise a tooth 42 acting as a positioning element for a machine connection element to facilitate an insertion of a machine connection element into the container connection element. In this view, also the inspection window 23 is visible. The blood treatment device is equipped with a sensor 43 configured to inspect a cap 6 present on the inner tube 18 through the inspection window 23.

In Fig. 10, the cap 6 is shown in an intact / closed state and in a broken / opened state. Depending on the state of the cap 6, a specific proportion of the field of vision of the sensor 43 through the inspection window is filled by at least one component of the cap 6. Thus, the sensor 43 can detect the presence of a cap 6 and preferably its state (opened or closed) through optical inspection through the inspection window 23.

Fig. 11a) illustrates a saddle-shaped rotation surface. As shown in Fig. 11a), a curved and / or rounded rotation surface 10 of a container attachment element according to an embodiment of the invention can have a saddle shape. In this configuration the saddle shaped rotation surface 10 would rest on a machine attachment element, e.g. a stud 11.

Fig. 11b) illustrates other possible geometrical configurations of rotation surfaces according to the present invention. As shown in Fig. 11b), a possible geometrical configurations is as a half-cylinder. The ground surface of the cylinder may also be oval and instead of a half-cylinder, a full cylinder may be used. The ground surface can have any other desirable shape, e.g. a bean or kidney-shape as shown in Fig. 11b), as longs as an at least partially rounded and / or curved rotation surface 10 results that is suitable and configured for rotation on a machine attachment element, as illustrated by the stud 11 in Fig. 11b).

The curved and / or rounded rotation surface 10 can be regarded as a contact surface between the container attachment element and the machine attachment element in a position in that the container attachment element is placed onto the machine attachment element to be held in place by gravity.

The curved and / or rounded rotation surface 10 can consist of or comprise a contact surface between the container attachment element and the machine attachment element in a position in that the container attachment element is placed onto the machine attachment element to be held in place by gravity.

Fig. 12 shows an example of a connection container comprising rounded surfaces 44 that are not rotation surfaces in the sense of the present disclosure. The surfaces 44 are not configured to allow for a rotation under the influence of gravity of the attachment element of the container if placed onto a machine attachment element (e.g. a stud 11).

Further, the surfaces 44 are not configured to be placed onto a corresponding machine attachment element of the blood treatment device in an attachment position to be held there by gravity. Instead, the connection portion shown in Fig. 12 is attached to a machine connection element such as a stud by inserting the stud into the openings 45 in a plug-and-socket-connection. The connection portion configured as shown in Fig. 12 is thus held at a machine attachment element by a form-fit-mechanism.

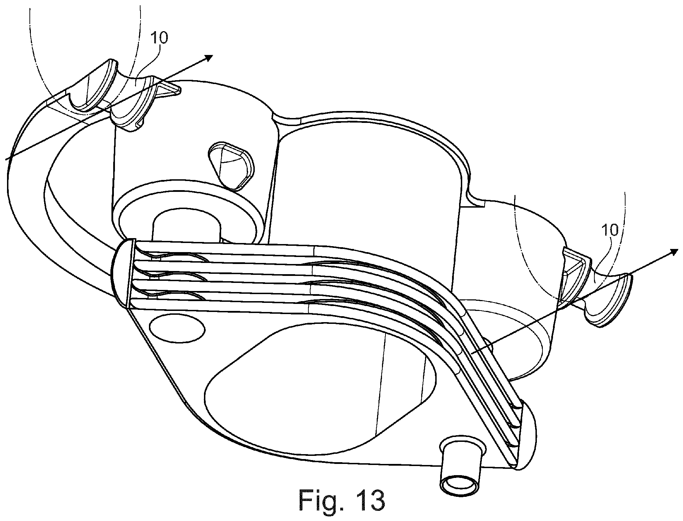

A further difference between a rounded and / or curved rotation surface as exemplified by the embodiments of the present invention and the surfaces 44 lies in the direction of the curvature of the surfaces. This is explained under reference to Figs. 12 and 13.

The surfaces 44 as shown in Fig. 12 are configured so that the circumference or curvature (as indicated by the dashed lines) defined by the surfaces 44 extends at an angle or at right angles to the direction (indicated by the arrows in Fig. 12) in that the container is moved to be attached to the blood treatment device by insertion of studs 11 into the openings 45.

In contrast to that, as shown in Fig. 13, the rotation surfaces 10 of an attachment element of a container according to the present invention are preferably configured so that the circumference or curvature (as indicated by the dashed lines) defined by the rotation surface 10 extends in the direction (as indicated by the arrows) in that the container is moved to be attached to the blood treatment device or parallel to that direction.

Figs 14a) to Fig. 14d) show schematic illustrations of container attachment elements comprising rotation surfaces 10 according to the present disclosure.

In the embodiments of Figs 14a) and Fig. 14b) no ribs 21 are shown and in the embodiments of Figs 14c) and Fig. 14d) ribs 21 are shown.

Fig. 14a) illustrates a schematic overview of a rotation surface 10 adjacent to that protrusions 22 are arranged that follow the circumference defined by the curvature of the rotation surface. The two protrusions 22 are arranged relative to each other at two opposing sides of the rotation surface 10 (front and back side in Fig. 14 a)).

At a side of the rotation surface 10 (right side in Fig. 14a)) opposite to the protrusions 22 (that are arranged at the left side in Fig. 14a)), a linking portion 46 is present linking the rotation surface 10 to the gripping portion 16. A linking point or connection point 47 in that the gripping portion 16 is joined to the linking portion 46 is offset from a center of gravity of the connection portion of the container.

Fig. 14b) illustrates a schematic overview of a rotation surface 10 adjacent to that protrusions 22 are arranged that project at right angles to the direction of gravity when the rotation surface 10 is placed onto a machine attachment element, e.g. a stud 11, in an attachment position.

Fig. 14 c) illustrates a view of the arrangement of Fig. 14a) when viewed from the left side in Fig. 14a) in that the two ribs 21 are visible. The two ribs 21 are arranged at two opposing sides (left and right side in Fig. 14c)) of the rotation surface 10. The rotation surface 10 thus forms the bottom surface of a groove defined between the two ribs 21.

Fig. 14d) illustrates a view of the arrangement of Fig. 14b) when viewed from the left side in Fig. 14b) in that the two ribs 21 are visible. The two ribs 21 are arranged at two opposing sides (left and right side in Fig. 14d)) of the rotation surface 10. The rotation surface 10 thus forms the bottom surface of a groove defined between the two ribs 21.

## Claims

1. Container (1) for concentrate, comprising:
- a hollow body (3) for containing concentrate,
- a connection portion (4) having at least one container connection element (7) configured to interact with a corresponding machine connection element (14) present on a blood treatment device to fluidically couple an inner volume of the hollow body (3) to the blood treatment device,
- two container attachment elements (6) arranged spaced apart from each other and configured to reversibly attach the container (1) to two corresponding machine attachment elements (11) present on a blood treatment device, **characterised in that**
- at least one of the two container attachment elements (6) comprises a rounded and / or curved rotation surface (10) configured to be placed onto a corresponding machine attachment element (11) of the blood treatment device in an attachment position to be held there by gravity and configured to bulge in the direction of gravity in the attachment position.

2. Container (1) according to claim 1, wherein both of the two container attachment elements (6) comprise a rounded and / or curved rotation surface (10).

3. Container (1) according to claim 1 or 2, wherein at least one container attachment element (6) comprises one or more protrusions (22) present at an end side of the rounded and / or curved rotation surface (10), wherein the one or more protrusions (22) project in a first direction and the container connection element (7) projects in a second direction that is oriented at an angle, preferably essentially at right angles, to the first direction, or the first direction projects essentially at right angles to a direction of gravity in the attachment position or the one or more protrusions (22) project along a curved, preferably circular, circumference defined by the rounded and / or curved rotation surface (10).

4. Container (1) according to one of the preceding claims, wherein at the rounded and / or curved rotation surface (10) of the container attachment element (6) at least one rib (21) is present to prevent lateral movement of the container (1) in the attachment position, wherein the at least one rib (21) preferably is arranged adjacent to the rounded and / or curved rotation surface (10) and / or extends along the rounded and / or curved rotation surface (10).

5. Container (1) according to one of the preceding claims, wherein the at least one container connection element (7) comprises an outer lumen and an inner lumen preferably arranged concentrically to the outer lumen, wherein an outer surface of a wall defining the inner lumen has a polygonal, preferably a hexagonal, geometry.

6. Container (1) according to claim 5, wherein on the wall defining the inner lumen of the at least one connection element a closing element, preferably a cap (9), is arranged wherein an inner contour of the closing element arranged on the wall defining the inner lumen matches the polygonal, preferably hexagonal, geometry of the outer surface of the wall defining the inner tube.

7. System comprising at least one container (1) according to one of claims 1 to 6 and at least one blood treatment device (2), the blood treatment device (2) comprising a housing (12),
two machine attachment elements (11) stationarily arranged at the housing (12) to that two corresponding container attachment elements (6) of a container (1) for concentrate are attachable, wherein at least one of the machine attachment elements (11) comprises a holding element onto that a rounded rotation surface (10) of a connector attachment element (7) is to be placed in the attachment position of the container (1) at the blood treatment device (2) in that the container (1) is held at the blood treatment (2) device by gravity; and two machine connection elements (14) pivotably arranged at a flap (13) pivotably arranged at the housing (12) and each fludically linked to a fluid path arranged in the flap (13), wherein the two machine connection elements (14) are configured to fluidically couple the blood treatment device (2) to the container (1) for concentrate by each being inserted into a corresponding container connection element (7), wherein the two machine connection elements (14) are rotationally movable in the same direction to be inserted into the corresponding container connection elements (7) upon a pivoting movement of the flap (13), wherein preferably the container (1) is reversibly attached by gravity to the blood treatment device (2) in the attachment position by means of the two container attachment elements (6) interacting with the two machine attachment elements (11) and / or the at least one machine connection element (14) is fluidically coupled to the at least one container connection element (7).

8. System according to claim 7, wherein
- at least one container connection element (7) comprises a wall defining an inner lumen, the wall having an outer surface with a hexagonal geometry, wherein a closing element is arranged on the wall defining the inner lumen and an inner contour of the closing element matches the hexagonal geometry of the outer surface of the wall defining the inner lumen of the container connection element (7), wherein the closing element comprises a closing portion comprising a plurality of plates arranged juxtaposed to each other separated by a plurality of intended breaking points; and
- at least one machine connection element (14) comprises a wall with a serrated leading edge comprising a plurality of teeth, wherein
- the wall of the machine connection element (14) and the closing element arranged on the wall defining the inner lumen of the container connection element (7) are arranged in such a way that upon connection of the machine connection element (14) with the container connection element (7), the teeth each exert pressure on one plate of the closing element so that each plate pivots around an edge of the hexagonal outer surface of the wall defining the inner lumen, thereby breaking at least one intended breaking point of the closing element and fluidically opening the closing element.

9. Method for connecting a container (1) for concentrate, preferably a container according of one of claims 1 to 6, to a blood treatment device (2), comprising the step:
- reversibly attaching the container (1) to the blood treatment device (2) by affixing two container attachment elements (6) to two corresponding machine attachment elements (11) by a combination of a rotational movement and an axial movement of the container and / or the two container attachment elements (6) relative to the blood treatment device (2) and / or the two machine attachment elements (11), wherein the rotational movement and the axial movement are performed at least partially simultaneously.

## Patentansprüche

1. Behälter (1) für Konzentrat, umfassend:
- einen Hohlkörper (3) zur Aufnahme von Konzentrat,
- einen Anschlussabschnitt (4) mit mindestens einem Behälteranschlusselement (7), das dazu ausgelegt ist, mit einem entsprechenden Maschinenanschlusselement (14), das an einer Blutbehandlungsvorrichtung vorhanden ist, zusammenzuwirken, um ein Innenvolumen des Hohlkörpers (3) fluidisch mit der Blutbehandlungsvorrichtung zu koppeln,
- zwei Behälterbefestigungselemente (6), die beabstandet zueinander angeordnet und dazu ausgelegt sind, den Behälter (1) reversibel an zwei entsprechenden Maschinenbefestigungselementen (11), die an einer Blutbehandlungsvorrichtung vorhanden sind, zu befestigen, **dadurch gekennzeichnet, dass**
- mindestens eines der zwei Behälterbefestigungselemente (6) eine abgerundete und / oder gekrümmte Drehfläche (10) umfasst, die dazu ausgelegt ist, in einer Befestigungsposition auf ein entsprechendes Maschinenbefestigungselement (11) der Blutbehandlungsvorrichtung aufgesetzt zu werden, um dort durch die Schwerkraft gehalten zu werden, und die dazu ausgelegt ist, sich in der Befestigungsposition in Richtung der Schwerkraft nach außen zu wölben.

2. Behälter (1) nach Anspruch 1, wobei beide der zwei Behälterbefestigungselemente (6) eine abgerundete und / oder gekrümmte Drehfläche (10) umfassen.

3. Behälter (1) nach Anspruch 1 oder 2, wobei mindestens ein Behälterbefestigungselement (6) einen oder mehrere Vorsprünge (22) umfasst, die an einer Stirnseite der abgerundeten und / oder gekrümmten Drehfläche (10) vorhanden sind, wobei der eine oder die mehreren Vorsprünge (22) in eine erste Richtung vorstehen und das Behälteranschlusselement (7) in eine zweite Richtung vorsteht, die in einem Winkel, vorzugsweise im Wesentlichen rechtwinklig, zur ersten Richtung orientiert ist, oder die erste Richtung im Wesentlichen rechtwinklig zu einer Richtung der Schwerkraft in der Befestigungsposition verläuft oder der eine oder die mehreren Vorsprünge (22) entlang eines gekrümmten, vorzugsweise kreisförmigen, Umfangs vorstehen, der durch die abgerundete und / oder gekrümmte Drehfläche (10) definiert ist.

4. Behälter (1) nach einem der vorhergehenden Ansprüche, wobei an der abgerundeten und / oder gekrümmten Drehfläche (10) des Behälterbefestigungselements (6) mindestens eine Rippe (21) vorhanden ist, um eine seitliche Bewegung des Behälters (1) in der Befestigungsposition zu verhindern, wobei die mindestens eine Rippe (21) vorzugsweise benachbart zu der abgerundeten und / oder gekrümmten Drehfläche (10) angeordnet ist und / oder sich entlang der abgerundeten und / oder gekrümmten Drehfläche (10) erstreckt.

5. Behälter (1) nach einem der vorhergehenden Ansprüche, wobei das mindestens eine Behälteranschlusselement (7) ein äußeres Lumen und ein inneres Lumen umfasst, das vorzugsweise konzentrisch zu dem äußeren Lumen angeordnet ist, wobei eine Außenfläche einer das innere Lumen definierenden Wand eine polygonale, vorzugsweise eine hexagonale, Geometrie aufweist.

6. Behälter (1) nach Anspruch 5, wobei an der das innere Lumen des mindestens einen Anschlusselements definierenden Wand ein Verschlusselement, vorzugsweise eine Kappe (9), angeordnet ist, wobei eine Innenkontur des auf der das innere Lumen definierenden Wand angeordneten Verschlusselements zu der polygonalen, vorzugsweise hexagonalen, Geometrie der Außenfläche der das innere Rohr definierenden Wand passt.

7. System, umfassend mindestens einen Behälter (1) nach einem der Ansprüche 1 bis 6 und mindestens eine Blutbehandlungsvorrichtung (2), wobei die Blutbehandlungsvorrichtung (2) ein Gehäuse (12) umfasst,
zwei Maschinenbefestigungselemente (11), die ortsfest am Gehäuse (12) angeordnet sind, so dass zwei entsprechende Behälterbefestigungselemente (6) eines Behälters (1) für Konzentrat befestigbar sind, wobei mindestens eines der Maschinenbefestigungselemente (11) ein Halteelement umfasst, auf das eine abgerundete Drehfläche (10) eines Anschlussbefestigungselements (7) in der Befestigungsposition des Behälters (1) an der Blutbehandlungsvorrichtung (2) aufgesetzt werden soll, so dass der Behälter (1) an der Blutbehandlungsvorrichtung (2) durch die Schwerkraft gehalten wird; und zwei Maschinenanschlusselemente (14), die schwenkbar an einer Klappe (13) angeordnet sind, die ihrerseits schwenkbar am Gehäuse (12) angeordnet ist, und die jeweils fluidisch mit einem in der Klappe (13) angeordneten Fluidpfad verbunden sind, wobei die zwei Maschinenanschlusselemente (14) dazu ausgelegt sind, die Blutbehandlungsvorrichtung (2) fluidisch mit dem Behälter (1) für Konzentrat zu koppeln, indem jedes in ein entsprechendes Behälteranschlusselement (7) eingeführt wird, wobei die zwei Maschinenanschlusselemente (14) in dieselbe Richtung drehbeweglich sind, um bei einer Schwenkbewegung der Klappe (13) in die entsprechenden Behälteranschlusselemente (7) eingeführt zu werden, wobei vorzugsweise der Behälter (1) in der Befestigungsposition mittels der zwei Behälterbefestigungselemente (6), die mit den zwei Maschinenbefestigungselementen (11) zusammenwirken, reversibel durch die Schwerkraft an der Blutbehandlungsvorrichtung (2) befestigt ist und / oder das mindestens eine Maschinenanschlusselement (14) fluidisch mit dem mindestens einen Behälteranschlusselement (7) gekoppelt ist.

8. System nach Anspruch 7, wobei
- mindestens ein Behälteranschlusselement (7) eine ein inneres Lumen definierende Wand umfasst, wobei die Wand eine Außenfläche mit einer hexagonalen Geometrie aufweist, wobei ein Verschlusselement auf der das innere Lumen definierenden Wand angeordnet ist und eine Innenkontur des Verschlusselements der hexagonalen Geometrie der Außenfläche der das innere Lumen definierenden Wand des Behälteranschlusselements (7) entspricht, wobei das Verschlusselement einen Verschlussabschnitt umfasst, der eine Mehrzahl von nebeneinander angeordneten Platten umfasst, die durch eine Mehrzahl von Sollbruchstellen voneinander getrennt sind; und
- mindestens ein Maschinenanschlusselement (14) eine Wand mit einer gezahnten Vorderkante umfasst, die eine Mehrzahl von Zähnen umfasst, wobei
- die Wand des Maschinenanschlusselements (14) und das auf der das innere Lumen des Behälteranschlusselements (7) definierenden Wand angeordnete Verschlusselement so angeordnet sind, dass beim Verbinden des Maschinenanschlusselements (14) mit dem Behälteranschlusselement (7) die Zähne jeweils Druck auf eine Platte des Verschlusselements ausüben, so dass jede Platte um eine Kante der hexagonalen Außenfläche der das innere Lumen definierenden Wand schwenkt, wodurch mindestens eine Sollbruchstelle des Verschlusselements aufbricht und das Verschlusselement fluidisch geöffnet wird.

9. Verfahren zum Verbinden eines Behälters (1) für Konzentrat, vorzugsweise eines Behälters nach einem der Ansprüche 1 bis 6, mit einer Blutbehandlungsvorrichtung (2), umfassend den Schritt:
- reversibles Befestigen des Behälters (1) an der Blutbehandlungsvorrichtung (2) durch Anbringen von zwei Behälterbefestigungselementen (6) an zwei entsprechenden Maschinenbefestigungselementen (11) durch eine Kombination aus einer Drehbewegung und einer axialen Bewegung des Behälters und / oder der zwei Behälterbefestigungselemente (6) relativ zu der Blutbehandlungsvorrichtung (2) und / oder den zwei Maschinenbefestigungselementen (11), wobei die Drehbewegung und die axiale Bewegung zumindest teilweise gleichzeitig ausgeführt werden.

## Revendications

1. Récipient (1) pour concentré, comprenant :
- un corps creux (3) destiné à contenir du concentré,
- une partie de raccordement (4) ayant au moins un élément de raccordement de récipient (7) configuré pour interagir avec un élément de raccordement de machine correspondant (14) présent sur un appareil de traitement du sang afin de coupler fluidiquement un volume intérieur du corps creux (3) à l'appareil de traitement du sang,
- deux éléments de fixation de récipient (6) disposés à distance l'un de l'autre et configurés pour fixer de manière réversible le récipient (1) à deux éléments de fixation de machine correspondants (11) présents sur un appareil de traitement du sang, **caractérisé en ce que**
- au moins l'un des deux éléments de fixation de récipient (6) comprend une surface de rotation arrondie et / ou courbe (10) configurée pour être placée sur un élément de fixation de machine correspondant (11) de l'appareil de traitement du sang dans une position de fixation afin d'y être maintenue par gravité et configurée pour faire saillie dans la direction de la gravité dans la position de fixation.

2. Récipient (1) selon la revendication 1, dans lequel les deux éléments de fixation de récipient (6) comprennent une surface de rotation arrondie et / ou courbe (10).

3. Récipient (1) selon la revendication 1 ou 2, dans lequel au moins un élément de fixation de récipient (6) comprend une ou plusieurs saillies (22) présentes sur une face d'extrémité de la surface de rotation arrondie et / ou courbe (10), la ou les saillies (22) faisant saillie dans une première direction et l'élément de raccordement de récipient (7) faisant saillie dans une seconde direction orientée selon un angle, de préférence essentiellement à angle droit, par rapport à la première direction, ou la première direction s'étendant essentiellement à angle droit par rapport à une direction de la gravité dans la position de fixation, ou la ou les saillies (22) faisant saillie le long d'une circonférence courbe, de préférence circulaire, définie par la surface de rotation arrondie et / ou courbe (10).

4. Récipient (1) selon l'une des revendications précédentes, dans lequel au niveau de la surface de rotation arrondie et / ou courbe (10) de l'élément de fixation de récipient (6), au moins une nervure (21) est présente pour empêcher un mouvement latéral du récipient (1) dans la position de fixation, la ou lesdites au moins une nervure (21) étant de préférence disposées adjacentes à la surface de rotation arrondie et / ou courbe (10) et / ou s'étendant le long de la surface de rotation arrondie et / ou courbe (10).

5. Récipient (1) selon l'une des revendications précédentes, dans lequel ledit au moins un élément de raccordement de récipient (7) comprend une lumière extérieure et une lumière intérieure, de préférence disposée concentriquement à la lumière extérieure, une surface extérieure d'une paroi définissant la lumière intérieure ayant une géométrie polygonale, de préférence hexagonale.

6. Récipient (1) selon la revendication 5, dans lequel sur la paroi définissant la lumière intérieure dudit au moins un élément de raccordement est disposé un élément de fermeture, de préférence un capuchon (9), une configuration intérieure de l'élément de fermeture disposé sur la paroi définissant la lumière intérieure correspondant à la géométrie polygonale, de préférence hexagonale, de la surface extérieure de la paroi définissant le tube intérieur.

7. Système comprenant au moins un récipient (1) selon l'une des revendications 1 à 6 et au moins un appareil de traitement du sang (2), l'appareil de traitement du sang (2) comprenant un boîtier (12),
deux éléments de fixation de machine (11) disposés de manière stationnaire sur le boîtier (12) de sorte que deux éléments de fixation de récipient correspondants (6) d'un récipient (1) pour concentré puissent y être fixés, au moins l'un des éléments de fixation de machine (11) comprenant un élément de retenue sur lequel une surface de rotation arrondie (10) d'un élément de fixation de raccordeur (7) doit être placée dans la position de fixation du récipient (1) sur l'appareil de traitement du sang (2), de sorte que le récipient (1) soit maintenu sur l'appareil de traitement du sang (2) par gravité ; et deux éléments de raccordement de machine (14) disposés de manière pivotante sur un volet (13) lui-même disposé de manière pivotante sur le boîtier (12) et chacun relié fluidiquement à un trajet de fluide disposé dans le volet (13), les deux éléments de raccordement de machine (14) étant configurés pour coupler fluidiquement l'appareil de traitement du sang (2) au récipient (1) pour concentré, chacun étant introduit dans un élément de raccordement de récipient correspondant (7), les deux éléments de raccordement de machine (14) étant mobiles en rotation dans la même direction pour être introduits dans les éléments de raccordement de récipient correspondants (7) lors d'un mouvement de pivotement du volet (13), le récipient (1) étant de préférence fixé de manière réversible par gravité à l'appareil de traitement du sang (2) dans la position de fixation au moyen des deux éléments de fixation de récipient (6) interagissant avec les deux éléments de fixation de machine (11) et / ou ledit au moins un élément de raccordement de machine (14) étant couplé fluidiquement audit au moins un élément de raccordement de récipient (7).

8. Système selon la revendication 7, dans lequel
- au moins un élément de raccordement de récipient (7) comprend une paroi définissant une lumière intérieure, la paroi ayant une surface extérieure avec une géométrie hexagonale, un élément de fermeture étant disposé sur la paroi définissant la lumière intérieure et une configuration intérieure de l'élément de fermeture correspondant à la géométrie hexagonale de la surface extérieure de la paroi définissant la lumière intérieure de l'élément de raccordement de récipient (7), l'élément de fermeture comprenant une partie de fermeture comprenant une pluralité de plaques disposées côte à côte, séparées les unes des autres par une pluralité de points de rupture prédéterminés ; et
- au moins un élément de raccordement de machine (14) comprend une paroi ayant un bord d'attaque dentelé comprenant une pluralité de dents, dans lequel
- la paroi de l'élément de raccordement de machine (14) et l'élément de fermeture disposé sur la paroi définissant la lumière intérieure de l'élément de raccordement de récipient (7) sont disposés de telle manière que, lors de la connexion de l'élément de raccordement de machine (14) avec l'élément de raccordement de récipient (7), les dents exercent chacune une pression sur une plaque de l'élément de fermeture de sorte que chaque plaque pivote autour d'une arête de la surface extérieure hexagonale de la paroi définissant la lumière intérieure, rompant ainsi au moins un point de rupture prédéterminé de l'élément de fermeture et ouvrant fluidiquement l'élément de fermeture.

9. Procédé de connexion d'un récipient (1) pour concentré, de préférence d'un récipient selon l'une des revendications 1 à 6, à un appareil de traitement du sang (2), comprenant l'étape consistant à :
- fixer de manière réversible le récipient (1) à l'appareil de traitement du sang (2) en fixant deux éléments de fixation de récipient (6) à deux éléments de fixation de machine correspondants (11) par une combinaison d'un mouvement de rotation et d'un mouvement axial du récipient et / ou des deux éléments de fixation de récipient (6) par rapport à l'appareil de traitement du sang (2) et / ou aux deux éléments de fixation de machine (11), le mouvement de rotation et le mouvement axial étant effectués au moins partiellement simultanément.
